Europäisches Patentamt

European Patent Office

Office européen des brevets

(11) Veröffentlichungsnummer: **0 265 853**
**A2**

# (12) EUROPÄISCHE PATENTANMELDUNG

(21) Anmeldenummer: 87115529.7

(22) Anmeldetag: 23.10.87

(51) Int. Cl.⁴: **C07C 41/06 , C07C 43/17**

(30) Priorität: 31.10.86 DE 3637124

(43) Veröffentlichungstag der Anmeldung:
04.05.88 Patentblatt 88/18

(84) Benannte Vertragsstaaten:
**BE CH DE FR GB IT LI NL**

(71) Anmelder: **HOECHST AKTIENGESELLSCHAFT**
**Postfach 80 03 20**
**D-6230 Frankfurt am Main 80(DE)**

(72) Erfinder: **Schwenk, Ulrich, Dr.**
**Robert-Koch-Strasse 209**
**D-8263 Burghausen(DE)**
Erfinder: **Streitberger, Horst**
**Josef-Hofmiller-Weg 9**
**D-8262 Altötting(DE)**
Erfinder: **Schulz, Hildegard**
**Graf-Lehnberger-Strasse 2**
**D-8343 Triftern(DE)**

(54) Verfahren zur Herstellung von Perfluoralkylethylallylethern.

(57) Es wird ein Verfahren zur Herstellung von Perfluoralkylethylallylethern beschrieben, bei dem Perfluoralkylethanol mit einem Allylhalogenid in Gegenwart eines Lösemittels und eines Alkalimetallhydroxids umgesetzt wird, wobei als Lösemittel Polyethylenglykoldialkylether und festes Alkalimetallhydroxid eingesetzt werden.

EP 0 265 853 A2

## Verfahren zur Herstellung von Perfluoralkylethylallylethern

Die Erfindung betrifft ein Verfahren zur Herstellung von Perfluoralkylethylallylethern durch Umsetzung von Perfluoralkylethanol mit einem Allylhalogenid in Gegenwart eines Lösemittels und eines Alkalimetallhydroxids zur Aufnahme der bei der Umsetzung freiwerdenden Halogenwasserstoffsäure.

Ein Verfahren zur Herstellung von Perfluoralkylethylallylethern ist aus der französischen Patentanmeldung 2 566 401 bekannt. Zur Veranschaulichung wird nachstehend die der Umsetzung zugrundeliegende Reaktionsgleichung angegeben ($R_f$ = Perfluoralkyl, X = Halogen):

$$R_fCH_2CH_2OH + CH_2=CH-CH_2X + NaOH\rightarrow$$
$$\rightarrow R_fCH_2CH_2OCH_2=CH=CH_2 + NaCl + H_2O$$

Bei der bekannten Umsetzung werden ausweislich aller Beispiele Ammoniumsalze, insbesondere das Hydrogensulfat von Tetrabutylammonium, als Phasentransferkatalysatoren eingesetzt. In der Beschreibung der Patentanmeldung wird zwar eine Reihe weiterer möglicher Katalysatoren genannt, wie tertiäre Amine, Polyamine, lineare Polyether, cyclische Polyether (Kronenether) und Kryptanden, all diesen wird jedoch offensichtlich keine Bedeutung beigemessen. Was die Menge an Phasentransferkatalysator betrifft, wird in der Druckschrift empfohlen, 0,1 bis 15 Mol-% einzusetzen, bezogen auf die Molmenge an OH-Gruppen im umzusetzenden Perfluoralkylethanol. Als Lösungsmittel werden in der Druckschrift Wasser und/oder mit Wasser nicht mischbare organische Flüssigkeiten wie Toluol, Nitrobenzol, Dichlorbenzol oder Chlormethylen beschrieben. Das bekannte Verfahren weist mehrere Nachteile auf. So läßt die Reinheit der angestrebten Perfluoralkylethylallylether, die nur bis zu 95 % beträgt, zu wünschen übrig. Es muß ferner ausweislich aller Beispiele ein relativ großer Überschuß an Allylchlorid eingesetzt werden, woraus weitere Nachteile resultieren.

Die Aufgabe der Erfindung besteht demnach darin, das eingangs genannte Verfahren zu verbessern. Es sollen insbesondere eine hohe Ausbeute und eine hohe Reinheit der angestrebten Ether erreicht werden.

Das erfindungsgemäße Verfahren zur Herstellung von Perfluoralkylethylallylethern durch Umsetzung von Perfluoralkylethanol mit einem Allylhalogenid in Gegenwart eines Lösemittels und eines Alkalimetallhydroxids zur Aufnahme der bei der Umsetzung freiwerdenden Halogenwasserstoffsäure, ist dadurch gekennzeichnet, daß Polyethylenglykoldialkylether als Lösemittel eingesetzt werden, daß diese Dialkylether in einer 0,2-bis 2fachen Menge, bezogen auf die Gewichtsmenge an umzusetzendem Perfluoralkylethanol, eingesetzt werden, und daß ferner das Alkalimetallhydroxid in fester Form eingesetzt wird (das heißt, in fester Form in der umzusetzenden Mischung vorliegt).

Erfindungsgemäß werden also Polyethylenglykoldialkylether als Lösemittel eingesetzt. Diese Polyglykoldiether entsprechen der nachstehenden Formel

$$RO(CH_2CH_2O)_yR'.$$

In der Formel sind R und R' bevorzugt die Methyl-, Ethyl-, Propyl-oder eine Butyl-Gruppe und besonders bevorzugt die Methyl-oder Ethyl-Gruppe. R und R' sind bevorzugt gleich. x ist eine Zahl von 3 bis 50, vorzugsweise 4 bis 25. Es kann ein Ether allein oder eine Mischung zweier oder mehrerer Ether eingesetzt werden. In der Regel wird es sich um eine Ethermischung handeln. Als Polyethylenglykoldialkylether werden demnach vorzugsweise Tetra-bis Pentaeicosaethylenglykoldimethyl-oder -diethylether, einzeln oder in Mischung, eingesetzt.

Die Menge an Polyethylenglykoldialkylether beträgt erfindungsgemäß das 0,2-bis 2fache, vorzugsweise das 0,2-bis 1fache, der Gewichtsmenge vom Perfluoralkylethanol, das zur Umsetzung mit Allylhalogenid eingesetzt wird.

Das Alkalimetallhydroxid, das vorzugsweise Kalium-oder Natriumhydroxid ist, wird erfindungsgemäß in fester Form eingesetzt, und zwar bevorzugt gepulvert oder gekörnt. Aufgrund seiner Funktion bei der in Rede stehenden Umsetzung wird mindestens 1 mol Alkalimetallhydroxid pro mol umzusetzendem Perfluoralkylethanol eingesetzt. Aus Zweckmäßigkeitsgründen werden erfindungsgemäß 1 bis 2 mol Alkalimetallhydroxid, vorzugsweise 1,2 bis 1,5 mol, pro mol Perfluoralkylethanol eingesetzt.

Was die Ausgangsverbindungen betrifft, ist das Allylhalogenid vorzugsweise Allylchlorid und das Perfluoralkylethanol vorzugsweise ein $C_2$-bis $C_{18}$-Perfluoralkylethanol, wobei die geradzahligen $C_4$-bis $C_{14}$-Vertreter besonders bevorzugt sind. Beim Perfluoralkyl-Rest handelt es sich also vorzugsweise um einen geradzahligen $C_4$-bis $C_{14}$-Rest, oder um eine Mischung zweier oder mehrerer solcher Reste. Die beiden Ausgangsverbindungen Perfluoralkylethanol und Allylhalogenid werden im molaren Verhältnis von 1 : 1 bis 5, vorzugsweise 1 : 1,5 bis 2,5, eingesetzt.

Die erfindungsgemäße Umsetzung wird bei einer Temperatur von 40 bis 100 °C, vorzugsweise 50 bis 80 °C, unter Druck oder drucklos durchgeführt. Es ist bevorzugt, die Umsetzung bei der angegebenen Temperatur und dem sich dabei einstellenden Druck durchzuführen. Die Reaktionszeit beträgt im allgemeinen 2 bis 8 h.

Nachstehend wird eine bevorzugte Ausführungsform des erfindungsgemäßen Verfahrens beschrieben.

Die Ausgangsverbindung Perfluoralkylethanol, der als Lösemittel fungierende Polyethylenglykoldialkylether und das Alkalimetallhydroxid in Pulver-oder Perlenform werden in einem Reaktionsgefäß vorgelegt. Gegebenenfalls können noch geringe Mengen eines Phasentransferkatalysators, etwa ein quartäres Ammonium-oder Phosphoniumsalz, zugesetzt werden. Die Mischung wird unter Rühren auf Reaktionstemperatur erhitzt, worauf unter weiterem Rühren das Allylhalogenid zudosiert wird. Nach der Zugabe des Allylhalogenids wird die Mischung noch solange bei der Reaktionstemperatur gehalten, bis die Umsetzung beendet ist. Es ist zweckmäßig, die vorgelegte Mischung nur auf eine solche Temperatur zu erhitzen, die im unteren Bereich der angegebenen Reaktionstemperatur von 40 bis 100 °C, vorzugsweise 50 bis 80 °C, liegt, bei dieser (niedrigeren) Temperatur das Allylhalogenid zuzugeben und erst nach der Zugabe auf eine höhere Reaktionstemperatur zu erhitzen und bei dieser höheren Temperatur die Umsetzung zu Ende zu führen. Die Aufarbeitung des Reaktionsproduktes zur Gewinnung des Perfluoralkylethylallylethers wird vorzugsweise nach einer der beiden folgenden Methoden durchgeführt. Nach der ersten Methode wird das Reaktionsprodukt zunächst vom gebildeten und in fester Form vorliegenden Alkalimetallsalz befreit, worauf die anfallende Flüssigkeitsmischung, bestehend im wesentlichen aus dem gebildeten Perfluoralkylethylallylether und dem eingesetzten Polyethylenglykoldialkylether, destillativ in diese beiden Verbindungen aufgetrennt wird. Nach der zweiten Methode wird das Reaktionsprodukt zunächst in Wasser aufgenommen, um das gebildete Alkalimetallsalz in Lösung zu bringen. Hierauf wird die organische Phase, bestehend im wesentlichen aus dem gebildeten Perfluoralkylethylallylether und dem eingesetzten Polyethylenglykoldialkylether von der wäßrigen Phase abgetrennt und wie bei der ersten Methode einer Destillation unterworfen, um den angestrebten Perfluoralkylethylallylether zu gewinnen.

Der nach den beschriebenen Aufarbeitungsmethoden erhaltene Perfluoralkylethylallylether ist im allgemeinen noch mehr oder weniger verunreinigt mit eingesetztem Perfluoralkylethanol. Es ist nun festgestellt worden, daß ein besonders reiner Perfluoralkylethylallylether dann erhalten wird, wenn der verunreinigte Ether in Gegenwart von Borsäure, $B(OH)_3$, auf Siedetemperatur erhitzt wird. Dabei bildet sich aus dem anwesenden Perfluoralkylethanol und der Borsäure Perfluoralkylethanolborat, eine Verbindung, die beträchtlich höher siedet als der Perfluoralkylethylallylether, der nun destillativ leicht und in sehr reiner Form abgetrennt werden kann. Es werden mindestens so viele mol Borsäure eingesetzt, wie mol Perfluoralkylethanol als Verunreinigung vorliegen und zu verestern sind. Es ist zweckmäßig, einen geringen Überschuß an Borsäure einzusetzen, und zwar einen Überschuß von etwa 0,1 bis 0,5 mol.

Das erfindungsgemäße Verfahren weist mehrere Vorteile auf. Der angestrebte Perfluoralkylethylallylether wird nicht nur in hoher Ausbeute, sondern auch in hoher Reinheit erhalten. Ferner wird der als Lösemittel eingesetzte Polyethylenglykoldialkylether praktisch vollständig wieder zurückgewonnen. Trotz des starken alkalischen Milieus infolge der Anwesenheit von festem Alkalimetallhydroxid, tritt überraschenderweise keine nennenswerte Abspaltung von Fluor aus dem Perfluoralkylethanol auf. Arbeitet man ohne die erfindungsgemäß einzusetzenden Lösungsmittel, so erhält man bei den in den nachstehenden Beispielen angegebenen Reaktionsbedingungen weniger als 10 % des angestrebten Ethers.

Mit den nachstehenden Beispielen wird das erfindungsgemäße Verfahren noch näher erläutert.

Beispiel 1

728 g (2 mol) $C_6F_{13}CH_2CH_2OH$, 160 g (4 mol)-pulverförmiges Natriumhydroxid und 370 g (das ist die 0,5fache Menge im Vergleich zur Menge an Perfluoralkylethanol) einer Mischung aus Polyethylenglykoldimethylethern der Formel $CH_2$-$(OCH_2CH_2)_n$ -$OCH_3$ mit n = 3 bis 8 (wobei die Ether mit n = 4 bis 6 die Hauptmenge darstellen; solche Mischungen sind unter dem Namen Polyethylenglykoldimethylether 250 bekannt) wurden in einem Reaktionsgefäß mit Tropftrichter und Kühler vorgelegt. Die Mischung wurde unter Rühren auf 40 °C erhitzt. In die so erhitzte Mischung wurden unter weiterem Rühren 306 g (4 mol) Allylchlorid zugetropft (exotherme Reaktion). Nach Beendigung der Allylchlorid-Zugabe wurde die Mischung auf 70 °C erhitzt und 5 Stunden lang bei dieser Temperatur gehalten. Nach dieser Zeit war die Umsetzung beendet. Das erhaltene Reaktionsprodukt wurde zur Abtrennung der festen Bestandteile (das sind gebildetes Natriumchlorid und überschüssiges Natriumhydroxid) filtriert. Das Filtrat wurde destilliert, um den angestrebten Perfluoralkylethylallylether abzutrennen. Nachdem der so erhaltene Ether noch verunreinigt war mit Perfluoralkylethanol (0,1 mol), wurde das Destillat mit 10 g (0,2 mol) Borsäure versetzt. Die Mischung wurde im Wasserstrahlvakuum auf Siedetemperatur erhitzt, wobei das entstehende Reaktionswasser (Veresterungswasser) abgetrennt wurde. Nach Abtrennung des Wassers wurde die angestrebte Etherverbindung überdestilliert.

Es wurden 751 g, das sind 93 Gew.-% der Theorie, an Perfluorhexylethylallylether, $C_6F_{13}CH_2CH_2OCH_2CH=CH_2$, mit einer Reinheit von mehr als 99 Gew.-% erhalten.

Beispiel 2

1268 g (2,5 mol) $C_8F_{17}CH_2CH_2OH$, 130 g (3,25 mol)perlenförmiges Natriumhydroxid und 400 g (das ist die 0,3fache Menge im Vergleich zur Menge an Perfluoralkylethanol) Polyethylenglykoldimethylether der Formel $CH_3$-$(OCH_2CH_2)_n$-$OCH_3$ mit n = ca. 11 (bekannt unter dem Namen Polyethylenglykoldimethylether 500) wurden in einem Reaktionsgefäß mit Tropftrichter und Kühler vorgelegt. Die Mischung wurde unter Rühren auf 40 °C erhitzt. In die so erhitzte Mischung wurden unter weiterem Rühren 249 g (3,25 mol) Allylchlorid zugetropft. Nach Beendigung der Allylchlorid-Zugabe wurde die Mischung auf 80 °C erhitzt und 3 h lang bei dieser Temperatur gehalten. Nach dieser Zeit war die Umsetzung beendet. Das erhaltene Reaktionsprodukt wurde bei 80 °C 2mal mit je 1,5 l Wasser gewaschen. Die gesammelten organischen Phasen stellten den angestrebten Ether dar, der aber noch mit eingesetztem Perfluoralkylethanol (0,1 mol) verunreinigt war. Der verunreinigte Ether wurde mit 15 g (0,25 mol) Borsäure versetzt. Die Mischung wurde im Wasserstrahlvakuum auf Siedetemperatur erhitzt, und das bei der Veresterungsreaktion gebildete Wasser abdestilliert. Nach Abtrennung des Wassers wurde die angestrebte Etherverbindung überdestilliert. Es wurden 1190 g, das sind 95 Gew.-% der Theorie, an Perfluoroctylethylallylether, $C_8F_{17}CH_2CH_2OCH_2CH=CH_2$, mit einer Reinheit von mehr als 99 Gew.-% erhalten.

Beispiel 3

Beispiel 2 wurde wiederholt mit dem Unterschied, daß anstelle der 400 g Polyethylenglykoldimethylether 500 650 g (das ist die 0,5fache Menge, bezogen auf die Menge an Perfluoralkylethanol) Polyethylenglykoldimethylether der Formel $CH_3$-$(OCH_2CH_2)_n$-$OCH_3$ mit n = ca. 22 eingesetzt wurde (dieser Dimethylether ist unter dem Namen Polyethylenglykoldimethylether 1000 bekannt). Es wurde eine Ausbeute und eine Reinheit wie in Beispiel 2 erreicht.

Beispiel 4

Beispiel 2 wurde wiederholt, mit dem Unterschied, daß anstelle von $C_8F_{17}CH_2CH_2OH$ die folgende Perfluoralkylethanol-Mischung eingesetzt wurde:
$C_6F_{13}CH_2CH_2OH$ (46 Gew.-%), $C_8F_{17}CH_2CH_2OH$ (32 Gew.-%), $C_{10}F_{21}CH_2CH_2OH$ (14 Gew.-%), $C_{12}F_{25}CH_2CH_2OH$ (6 Gew.-%) und $C_{14}F_{29}CH_2CH_2OH$ (2 Gew.-%).
Es wurde eine Ausbeute und eine Reinheit wie in Beispiel 2 erreicht.

Beispiel 5

Beispiel 1 wurde wiederholt, mit dem Unterschied, daß nicht 370 g, sondern 728 g Polyethylenglykoldimethylether 250 eingesetzt wurden (das ist die einfache Menge an Lösemittel, bezogen auf die Menge an eingesetztem Perfluoralkylethanol).

Es wurden die Ausbeute und die Reinheit des Beispiels 1 erreicht.

**Ansprüche**

1. Verfahren zur Herstellung von Perfluoralkylethylallylethern durch Umsetzung von Perfluoralkylethanol mit Allylhalogenid in Gegenwart eines Lösemittels und eines Alkalimetallhydroxids zur Aufnahme der bei der Umsetzung freiwerdenden Halogenwasserstoffsäure, dadurch gekennzeichnet, daß Polyethylenglykoldialkylether als Lösemittel eingesetzt werden, daß diese Dialkylether in einer 0,2-bis 2fachen Menge, bezogen auf die Gewichtsmenge an umzusetzendem Perfluoralkylethanol, eingesetzt werden, und daß ferner das Alkalimetallhydroxid in fester Form eingesetzt wird.

2. Verfahren nach Anspruch 1, dadurch gekennzeichnet, daß als Polyethylenglykoldialkylether Tetra-bis Pentaeicosaethylenglykoldimethyl-oder -diethylether eingesetzt werden.

3. Verfahren nach Anspruch 1 oder 2, dadurch gekennzeichnet, daß die Polyethylenglykoldialkylether in einer 0,2-bis 1fachen Menge, bezogen auf die Gewichtsmenge an umzusetzendem Perfluoralkylethanol, eingesetzt werden.

4. Verfahren nach einem oder mehreren der Ansprüche 1 bis 3, dadurch gekennzeichnet, daß die Umsetzung bei einer Temperatur von 40 bis 100 °C und dem sich bei dieser Temperatur einstellenden Druck durchgeführt wird.

5. Verfahren nach einem oder mehreren der Ansprüche 1 bis 4, dadurch gekennzeichnet, daß der nach destillativer Aufarbeitung des Reaktionsproduktes erhaltene Perfluoralkylethylallylether in Gegenwart von Borsäure erhitzt wird, um als Verunreinigung vorhandenes Perfluoralkylethanol in das höhersiedende Perfluoralkylethanolborat umzuwandeln, und daß aus der Mischung Perfluoralkylethanolborat und Perfluoralkylethylallylether der angestrebte Ether destillativ abgetrennt wird.